# EUROPÄISCHE PATENTSCHRIFT

(11) **EP 1 368 480 B1**
(45) Veröffentlichungstag und Bekanntmachung des Hinweises auf die Patenterteilung: **26.04.2006**
(21) Anmeldenummer: 02719954.6
(22) Anmeldetag: 28.02.2002
(51) Int. Cl.: C12N 15/54

(54) **VERFAHREN ZUR VERÄNDERUNG DES GENOMS VON GRAM-POSITIVEN BAKTERIEN MIT EINEM NEUEN KONDITIONAL NEGATIV DOMINANTEN MARKERGEN**
METHOD OF MODIFYING THE GENOME OF GRAM-POSITIVE BACTERIA BY MEANS OF A NOVEL CONDITIONALLY NEGATIVE DOMINANT MARKER GENE
PROCEDE DE MODIFICATION DU GENOME DE BACTERIES GRAM POSITIF AU MOYEN D'UN NOUVEAU GENE MARQUEUR A DOMINANTE CONDITIONNELLEMENT NEGATIVE

(30) Priorität: 01.03.2001 DE 10109996
(43) Veröffentlichungstag der Anmeldung: 10.12.2003
(73) Patentinhaber: BASF Aktiengesellschaft, 67056 Ludwigshafen (DE)
(72) Erfinder: POMPEJUS, Markus, 67251 Freinsheim (DE); KRÖGER, Burkhard, 67117 Limburgerhof (DE); SCHRÖDER, Hartwig, 69226 Nussloch (DE); ZELDER, Oskar, 67346 Speyer (DE)
(86) Internationale Anmeldenummer: PCT/EP2002/002133
(87) Internationale Veröffentlichungsnummer: WO 2002/070685

(56) Entgegenhaltungen:
- WO-A-91/00913
- SCHAEFER A ET AL: "SMALL MOBILIZABLE MULTI-PURPOSE CLONING VECTORS DERIVED FROM THE ESCHERICHIA COLI PLASMIDS PK18 AND PK19: SELECTION OF DEFINED DELETIONS IN THE CHROMOSOME OF CORYNEBACTERIUM GLUTAMICUM" GENE, ELSEVIER BIOMEDICAL PRESS. AMSTERDAM, NL, Bd. 145, Nr. 145, 1994, Seiten 69-73, XP001093898 ISSN: 0378-1119 in der Anmeldung erwähnt
- TANG L B ET AL: "ISOLATION AND CHARACTERIZATION OF LEVANSUCRASE-ENCODING GENE FROM BACILLUS AMYLOLIQUEFACIENS" GENE, ELSEVIER BIOMEDICAL PRESS. AMSTERDAM, NL, Bd. 96, 1990, Seiten 89-93, XP000651635 ISSN: 0378-1119 in der Anmeldung erwähnt
- BRAMUCCI M.G.; NAGARAJAN V.: 'DIRECT SELECTION OF CLONED DNA IN BACILLUS SUBTILIS BASED ON SUCROSE-INDUCED LETHALITY' APPLIED AND ENVIRONMENTAL MICROBIOLOGY, WASHINGTON,DC, US Bd. 62, Nr. 11, 01 November 1996, Seiten 3948 - 3953, XP000651614
- SELBITSCHKA W.; NIEMANN S.; PUEHLER A.: 'CONSTRUCTION OF GENE REPLACEMENT VECTORS FOR GRAM - BATERIA USING A GENETICALLY MODIFIED SACRB GENE AS A POSITIVE SELECTION MARKER' APPLIED MICROBIOLOGY AND BIOTECHNOLOGY, SPRINGER VERLAG, BERLIN, DE Bd. 38, Nr. 5, 01 Februar 1993, Seiten 615 - 618, XP000579453

### SEQUENCE LISTING

<110> BASF Aktiengesellschaft
<120> Verfahren zur Ver,,nderung des Genoms von gram-positiven Bakterein mit einem neuen konditional negativen Markergen
<130> PF000052287
<140> EP20020719954
   <141> 2002-02-28
<150> DE20011009996
   <151> 2001-03-01
<160> 4
<170> PatentIn Ver. 2.0
<110> 1
   <111> 54
   <112> DNA
   <113> Artificial Sequence
<220>
   <223> Description of Artificial Sequence:synthetic primer
<400> 1
<210> 2
   <211> 27
   <212> DNA
   <213> Artificial Sequence
<220>
   <223> Description of Artificial Sequence:synthetic primer
<400> 2
   actagtttag ttgactgtca gctgtcc 27
<210> 3
   <211> 32
   <212> DNA
   <213> Artificial Sequence
<220>
   <223> Description of Artificial Sequence:synthetic primer
<400> 3
   ggtaccgttc tggcaaatat tctgaaatga gc 32
<210> 4
   <211> 30
   <212> DNA
   <213> Artificial Sequence
<220>
   <223> Description of Artificial Sequence:synthetic primer
<400> 4
   gcggccgctt ctgtttcctg tgtgaaattg 30

## Beschreibung

Die Erfindung betrifft ein neues Verfahren zur Veränderung des Genoms von gram-positiven Bakterien, diese Bakterien und neue Vektoren. Insbesondere betrifft die Erfindung ein Verfahren zur Veränderung von Corynebacterien oder Brevibacterien mit Hilfe eines neuen in den Bakterien konditional negativ dominant wirkenden Markergens.

Corynebacterium glutamicum ist ein gram-positives, aerobes Bakterium, das (wie auch andere Corynebakterien, d.h. Corynebacterium und Brevibacterium - Arten) in der Industrie für die Produktion einer Reihe von Feinchemikalien, und auch zum Abbau von Kohlenwasserstoffen und zur Oxidation von Terpenoiden verwendet wird (Zur Übersicht siehe z.B. Liebl (1992) "The Genus Corynebacterium", in: The Procaryotes, Volume II, Balows, A. et al., eds. Springer).

Aufgrund der Verfügbarkeit von Klonierungsvektoren zur Verwendung in Corynebakterien und Techniken zur genetischen Manipulation von *C. glutamicum* und verwandten *Corynebacterium* und *Brevibacterium-*Arten (siehe z.B. Yoshihama et al., J. Bacteriol. 162 (1985) 591-597; Katsumata et al., J. Bacteriol. 159 (1984) 306-311; und Santamaria et al. J. Gen. Microbiol. 130 (1984) 2237-2246) ist es möglich, diese Organismen genetisch zu verändern (beispielsweise durch Überexpression von Genen) um sie beispielsweise als Produzenten von einer oder mehreren Feinchemikalien besser und effizienter zu machen.

Die Verwendung von Plasmiden, die in Corynebakterien replizieren können ist dabei eine gut etablierte Technik, die dem Fachmann bekannt ist, breit angewendet wird und mehrfach in der Literatur dokumentiert ist (siehe z.B. Deb, J.K et al. (1999) FEMS Microbiol. Lett. 175, 11-20).

Es ist ebenfalls möglich, Corynebakterien dadurch genetisch zu verändern, dass die DNA-Sequenz des Genoms modifiziert wird. Es können DNA-Sequenzen in das Genom eingebracht werden (neu eingebracht und/oder vorhandene Sequenzen in weiteren Kopien eingebracht werden), es können auch DNA-Sequenzabschnitte aus dem Genom entfernt werden (z.B. Gene oder Teile von Genen), es können aber auch Sequenzaustausche (z.B. Basenaustausche) im Genom durchgeführt werden.

Die Veränderung des Genoms kann dadurch erreicht werden, dass DNA in die Zelle eingebracht wird, die vorzugsweise nicht in der Zelle repliziert und dass diese eingebrachte DNA mit genomischer Wirts-DNA rekombiniert und so die genomische DNA verändert. Diese Vorgehensweise ist beispielsweise beschrieben in van der Rest, M.E. et al. (1999) Appl. Microbiol. Biotechnol. 52, 541-545 und Referenzen darin.

Es ist vorteilhaft, den verwendeten Transformationsmarker (wie z.B. ein Antibiotikaresistenzgen) wieder entfernen zu können, da dieser Marker dann bei weiteren Transformationsexperimenten wieder verwendet werden kann. Eine Möglichkeit dies durchzuführen, ist der Einsatz eines konditional negativ dominant wirkenden Markergens.

Unter einem konditional negativ dominant wirkenden Markergen ist ein Gen zu verstehen, dass unter bestimmten Bedingungen nachteilig (z.B. toxisch) für den Wirt ist, unter anderen Bedingungen aber keine negativen Auswirkungen auf den das Gen tragenden Wirt hat. Ein Literatur-bekanntes Beispiel ist das URA3 Gen aus Hefen oder Pilzen, ein essentielles Gen der Pyrimidinbiosynthese, das jedoch für den Wirt nachteilig ist, wenn im Medium die Chemikalie 5-Fluoro-Orotsäure vorliegt (siehe beispielsweise DE19801120, Rothstein, R. (1991) Methods in Enzymology 194, 281-301).

Die Verwendung eines konditional negativ dominant wirkenden Markergens zur Entfernung von DNA-Sequenzen (beispielsweise der verwendeten Transformationsmarker und/oder von Vektorsequenzen und anderer Sequenzabschnitte), auch "*pop-out*" genannt ist beispielsweise beschrieben in Schäfer et al. (1994) Gene 14, 69-73 oder in Rothstein, R. (1991) Methods in Enzymology 194, 281-301.

Das Gen sacB aus Bacillus subtilis kodiert für das Enzym Levansucrase (EC 2.4.1.10) und wurde beschrieben in Steinmetz, M. et al. (1983) Mol. Gen. Genet. 191, 138-144, und Steinmetz, M. et al. (1985) Mol. Gen. Genet. 200, 220-228. Es ist bekannt (Gay, P. et al. (1985) J. Bacteriology 164, 918-921, Schäfer et al. (1994) Gene 145 69-73, EP0812918, EP0563527, EP0117823), dass sich das sacB Gen aus Bacillus subtilis als konditional negativ dominant wirkendes Markergen eignet. Dieses Selektionsverfahren beruht darauf, daß Zellen, die das sacB Gen tragen, nicht in Anwesenheit von 5% Saccharose wachsen können. Erst der Verlust oder die Inaktivierung der Levansucrase führt zum Wachstum von Zellen. Die Sensitivität bestimmter gram-positiver Bakterien, die das sacB Gen aus Bacillus subtilis exprimieren gegenüber 10% Saccharose wurde dann von Jäger, W. et al. (1992) J. Bacteriology 174, 5462-5465 beschrieben werden. In Corynebacterium glutamicum wurde außerdem gezeigt, daß mit dem sacB Gan aus B. subtilis eine Selektion auf Gendisruptionen oder.ein allelischer Austausch durch homologe Rekombination durchgeführt werden kann (Schäfer et al. (1994) Gene 145, 69-73).

Es wurde nun gefunden, dass sich das sacB Gen aus Bacillus amyloliquefaciens (Tang et al. (1990) Gene 96, 89-93) überraschender Weise besonders gut für den Einsatz als konditional negativ dominant wirkendes Markergen in Corynebakterien eignet. Die Selektionsfähigkeit mittels sacB hängt von der Expressionseffizienz des Gens im heterologen Wirtsorganismus ab. Die gute Expressionseffizienz des sacB Gens aus B. amyloliquefaciens macht dieses Gen zu einem bevorzugt verwendeten Gen.

Die Erfindung offenbart eine neue und einfache Methode zur Veränderung von genomischen Sequenzen in Corynebakterien unter Verwendung des sacB Gens aus Bacillus amyloliquefaciens als neues konditional negativ dominant wirkendes Markergen. Dies können genomische Integrationen von Nukleinsäuremolekülen (beispielsweise komplette Gene), Disruptionen (beispielsweise Deletionen oder integrative Disruptionen) und Sequenzveränderungen (beispielsweise einfache oder mehrfache Punktmutationen, komplette Gen-Austauscher) sein. Bevorzugte Disruptionen sind solche die zu einer Reduzierung von Nebenprodukten des gewünschten Fermentationsproduktes führen, bevorzugte Integrationen sind solche, die einen gewünschten Metabolismus zu einem Fermentationsprodukt verstärken und/oder 'Flaschenhälse' abmindern oder aufheben (de-bottlenekking). Bei Sequenzveränderungen sind entsprechende metabolische Anpassungen bevorzugt. Bei dem Fermentationsprodukt handelt es sich bevorzugt um eine Feinchemikalie.

Die Erfindung betrifft insbesondere einen Plasmidvektor, der in Bakterienstämmen der Gattung, Brevibacterium oder Corynebacterium nicht repliziert, mit folgenden Komponenten:
a) einen Replikationsursprung (*origin of replication*) für *E. coli,*
b) einen oder mehrere genetische Marker,
c) optional einen Sequenzabschnitt, der den Transfer von DNA ermöglicht, insbesondere durch Konjugation (mob),
d) einen Sequenzabschnitt, der homolog zu Sequenzen der Bakterienstämme der Gattung Brevibacterium oder Corynebacterium ist und in diesen Bakterienstämmen homologe Rekombination vermittelt,
e) das sacB Gen aus *B. amyloliquefaciens* unter der Kontrolle eines Promotors.

Unter Zielorganismus ist dabei der Organismus zu verstehen, dessen genomische Sequenz verändert werden soll.

Die Erfindung betrifft außerdem ein Verfahren zur *marker-freien Mutagenese* in *gram-positiven Bakterien-Stamm* aus der Gattung Brevibacterium oder Corynebacterium umfassend folgende Schritte:
a) zur Verfügung stellen eines Vektors wie oben angegeben,
b) Transfer des Vektors in ein gram-positives Bakterium aus der Gattung Brevibacterium oder Corynebacterium
c) selektieren auf einen oder mehrere genetische Marker
d) selektieren eines oder mehrerer Klone von transfizierten gram-positiven Bakterien, indem die transfizierten Klone in einem Saccharose-haltigem Medium kultiviert werden
und ein Bakterium, erhältlich nach diesem Verfahren bis zu Schritt c).

Der Promotor ist bevorzugt heterolog zu *B. amyloliquefaciens* und ist insbesondere aus E. coli oder C. glutamicum und weiterhin insbesondere der tac-Promotor.

Unter den Sequenzen, die im Zielorganismus ausgetauscht werden, sind insbesondere solche, die die Ausbeuten bei der Erhöhung der Produktion von Feinchemikalien bewirken. Beispiele für solche Gene sind in WO 01/0842, 843 & 844, WO 01/0804 & 805, WO 01/2583 angegeben.

Der Transfer von DNA in den Zielorganismus wird insbesondere durch Konjugation oder Elektroporation ermöglicht. DNA, die durch Konjugation in den Zielorganismus transferiert werden soll, enthält spezielle Sequenzabschnitte, die dies ermöglichen. Solche sogenannten mob Sequenzen und ihre Verwendung sind beispielsweise beschrieben in Schäfer, A. et al. (1991) J. Bacteriol. 172, 1663-1666.

Unter genetischer Marker wird eine selektionierbare Eigenschaft verstanden. Dies sind bevorzugt Antibiotika Resistenzen, insbesondere eine Resistenz gegen Kanamycin, Chloramphenicol, Tetrazyklin oder Ampicillin.

Unter Saccharose-haltigem Medium wird insbesondere ein Medium mit mindestens 5 % und höchstens 10 % (Gew.) Saccharose verstanden.

Unter Zielorganismus ist der Organismus zu verstehen, der genetisch durch die erfindungsgemäßen Verfahren modifiziert werden soll. Dies sind bevorzugt gram-positive Bakterien zu verstehen, insbesondere Bakterien-Stämme aus der Gattung Brevibacterium oder Corynebacterium. Unter Corynebakterien im Sinne der Erfindung werden Corynebacterium-Arten, Brevibacterium-Arten und Mycobacterium-Arten verstanden. Bevorzugt sind Corynebacterium-Arten und Brevibacterium-Arten. Beispiele für Corynebacterium-Arten und Brevibacterium-Arten sind: Brevibacterium brevis, Brevibacterium lactofermentum, Corynebacterium ammoniagenes, Corynebacterium glutamicum, Corynebacterium diphtheriae, Corynebacterium lactofermentum. Beispiele für Mycobacterium-Arten sind: Mycobacterium tuberculosis, Mycobacterium leprae, Mycobacterium bovis, Mycobacterium smegmatis.

Insbesondere sind folgende in der Tabelle angegebenen Stämme bevorzugt:

**Tabelle: Corynebacterium und Brevibacterium Stämme:**

| Genus | species | ATCC | FERM | NRRL | CECT | NCIMB | CBS |
|---|---|---|---|---|---|---|---|
| Brevibacterium | ammoniagenes | 21054 | | | | | |
| Brevibacterium | ammoniagenes | 19350 | | | | | |
| Brevibacterium | ammoniagenes | 19351 | | | | | |
| Brevibacterium | ammoniagenes | 19352 | | | | | |
| Brevibacterium | ammoniagenes | 19353 | | | | | |
| Brevibacterium | ammoniagenes | 19354 | | | | | |
| Brevibacterium | ammoniagenes | 19355 | | | | | |
| Brevibacterium | ammoniagenes | 19356 | | | | | |
| Brevibacterium | ammoniagenes | 21055 | | | | | |
| Brevibacterium | ammoniagenes | 21077 | | | | | |
| Brevibacterium | ammoniagenes | 21553 | | | | | |
| Brevibacterium | ammoniagenes | 21580 | | | | | |
| Brevibacterium | ammoniagenes | 39101 | | | | | |
| Brevibacterium | butanicum | 21196 | | | | | |
| Brevibacterium | divaricatum | 21792 | P928 | | | | |
| Brevibacterium | flavum | 21474 | | | | | |
| Brevibacterium | flavum | 21129 | | | | | |
| Brevibacterium | flavum | 21518 | | | | | |
| Brevibacterium | flavum | | | B11474 | | | |
| Brevibacterium | flavum | | | B11472 | | | |
| Brevibacterium | flavum | 21127 | | | | | |
| Brevibacterium | flavum | 21128 | | | | | |
| Brevibacterium | flavum | 21427 | | | | | |
| Brevibacterium | flavum | 21475 | | | | | |
| Brevibacterium | flavum | 21517 | | | | | |
| Brevibacterium | flavum | 21528 | | | | | |
| Brevibacterium | flavum | 21529 | | | | | |
| Brevibacterium | flavum | | | B11477 | | | |
| Brevibacterium | flavum | | | B11478 | | | |
| Brevibacterium | flavum | 21127 | | | | | |
| Brevibacterium | flavum | | | B11474 | | | |
| Brevibacterium | heaiii | 15527 | | | | | |
| Brevibacterium | ketoglutamicum | 21004 | | | | | |
| Brevibacterium | keiogiuiamicum | 21089 | | | | | |
| Brevibacterium | ketosoreductum | 21914 | | | | | |
| Brevibacterium | lactofermentum | | | | 70 | | |
| Brevibacterium | lactofermentum | | | | 74 | | |
| Brevibacterium | lactofermentum | | | | 77 | | |
| Brevibacterium | lactofermentum | 21798 | | | | | |
| Brevibacterium | lactofermentum | 21799 | | | | | |
| Brevibacterium | lactofermentum | 21800 | | | | | |
| Brevibacterium | lactofermentum | 21801 | | | | | |
| Brevibacterium | lactofermentum | | | B11470 | | | |
| Brevibacterium | lactofermentum | | | B11471 | | | |
| Brevibacterium | lactofermentum | 21086 | | | | | |
| Brevibacterium | lactofermentum | 21420 | | | | | |
| Brevibacterium | lactofermentum | 21086 | | | | | |
| Brevibacterium | lactofermentum | 31269 | | | | | |
| Brevibacterium | linens | 9174 | | | | | |
| Brevibacterium | linens | 19391 | | | | | |
| Brevibacterium | linens | 8377 | | | | | |
| Brevibacterium | paraffinolyticum | | | | | 11160 | |
| Brevibacterium | spec. | | | | | | 717.73 |
| Brevibacterium | spec. | | | | | | 717.73 |
| Brevibacterium | spec. | 14604 | | | | | |
| Brevibacterium | spec. | 21860 | | | | | |
| Brevibacterium | spec. | 21864 | | | | | |
| Brevibacterium | spec. | 21865 | | | | | |
| Brevibacterium | spec. | 21866 | | | | | |
| Brevibacterium | spec. | 19240 | | | | | |
| Corynebacterium | acetoacidophilum | 21476 | | | | | |
| Corynebacterium | acetoacidophilum | 13870 | | | | | |
| Corynebacterium | acetoglutamicum | | | B11473 | | | |
| Corynebacterium | acetoglutamicum | | | B11475 | | | |
| Corynebacterium | acetoglutamicum | 15806 | | | | | |
| Corynebacterium | acetoglutamicum | 21491 | | | | | |
| Corynebacterium | acetoglutamicum | 31270 | | | | | |
| Corynebacterium | acetophilum | | | B3671 | | | |
| Corynebacterium | ammoniagenes | 6872 | | | | | |
| Corynebacterium | ammoniagenes | 15511 | | | | | |
| Corynebacterium | fujiokense | 21496 | | | | | |
| Corynebacterium | glutamicum | 14067 | | | | | |
| Corynebacterium | glutamicum | 39137 | | | | | |
| Corynebacterium | glutamicum | 21254 | | | | | |
| Corynebacterium | glutamicum | 21255 | | | | | |
| Corynebacterium | glutamicum | 31830 | | | | | |
| Corynebacterium | glutamicum | 13032 | | | | | |
| Corynebacterium | glutamicum | 14305 | | | | | |
| Corynebacterium | glutamicum | 15455 | | | | | |
| Corynebacterium | glutamicum | 13058 | | | | | |
| Corynebacterium | glutamicum | 13059 | | | | | |
| Corynebacterium | glutamicum | 13060 | | | | | |
| Corynebacterium | glutamicum | 21492 | | | | | |
| Corynebacterium | glutamicum | 21513 | | | | | |
| Corynebacterium | glutamicum | 21526 | | | | | |
| Corynebacterium | glutamicum | 21543 | | | | | |
| Corynebacterium | glutamicum | 13287 | | | | | |
| Corynebacterium | glutamicum | 21851 | | | | | |
| Corynebacterium | glutamicum | 21253 | | | | | |
| Corynebacterium | glutamicum | 21514 | | | | | |
| Corynebacterium | glutamicum | 21516 | | | | | |
| Corynebacterium | glutamicum | 21299 | | | | | |
| Corynebacterium | glutamicum | 21300 | | | | | |
| Corynebacterium | glutamicum | 39684 | | | | | |
| Corynebacterium | glutamicum | 21488 | | | | | |
| Corynebacterium | glutamicum | 21649 | | | | | |
| Corynebacterium | glutamicum | 21650 | | | | | |
| Corynebacterium | glutamicum | 19223 | | | | | |
| Corynebacterium | glutamicum | 13869 | | | | | |
| Corynebacterium | glutamicum | 21157 | | | | | |
| Corynebacterium | glutamicum | 21158 | | | | | |
| Corynebacterium | glutamicum | 21159 | | | | | |
| Corynebacterium | glutamicum | 21355 | | | | | |
| Corynebacterium | glutamicum | 31808 | | | | | |
| Corynebacterium | glutamicum | 21674 | | | | | |
| Corynebacterium | glutamicum | 21562 | | | | | |
| Corynebacterium | glutamicum | 21563 | | | | | |
| Corynebacterium | glutamicum | 21564 | | | | | |
| Corynebacterium | glutamicum | 21565 | | | | | |
| Corynebacterium | glutamicum | 21566 | | | | | |
| Corynebacterium | glutamicum | 21567 | | | | | |
| Corynebacterium | glutamicum | 21568 | | | | | |
| Corynebacterium | glutamicum | 21569 | | | | | |
| Corynebacterium | glutamicum | 21570 | | | | | |
| Corynebacterium | glutamicum | 21571 | | | | | |
| Corynebacterium | glutamicum | 21572 | | | | | |
| Corynebacterium | glutamicum | 21573 | | | | | |
| Corynebacterium | glutamicum | 21579 | | | | | |
| Corynebacterium | glutamicum | 19049 | | | | | |
| Corynebacterium | glutamicum | 19050 | | | | | |
| Corynebacterium | glutamicum | 19051 | | | | | |
| Corynebacterium | glutamicum | 19052 | | | | | |
| Corynebacterium | glutamicum | 19053 | | | | | |
| Corynebacterium | glutamicum | 19054 | | | | | |
| Corynebacterium | glutamicum | 19055 | | | | | |
| Corynebacterium | glutamicum | 19056 | | | | | |
| Corynebacterium | glutamicum | 19057 | | | | | |
| Corynebacterium | glutamicum | 19058 | | | | | |
| Corynebacterium | glutamicum | 19059 | | | | | |
| Corynebacterium | glutamicum | 19060 | | | | | |
| Corynebacterium | glutamicum | 19185 | | | | | |
| Corynebacterium | glutamicum | 13286 | | | | | |
| Corynebacterium | glutamicum | 21515 | | | | | |
| Corynebacterium | glutamicum | 21527 | | | | | |
| Corynebacterium | glutamicum | 21544 | | | | | |
| Corynebacterium | glutamicum | 21492 | | | | | |
| Corynebacterium | glutamicum | | | B8183 | | | |
| Corynebacterium | glutamicum | | | B8182 | | | |
| Corynebacterium | glutamicum | | | B12416 | | | |
| Corynebacterium | glutamicum | | | B12417 | | | |
| Corynebacterium | glutamicum | | | B12418 | | | |
| Corynebacterium | glutamicum | | | B11476 | | | |
| Corynebacterium | glutamicum | 21608 | | | | | |
| Corynebacterium | lilium | | F973 | | | | |
| Corynebacterium | nitrilophilus | 21419 | | | | 11594 | |
| Corynebacterium | spec. | | P4445 | | | | |
| Corynebacterium | spec. | | P4446 | | | | |
| Corynebacterium | spec. | 31088 | | | | | |
| Corynebacterium | spec. | 31089 | | | | | |
| Corynebacterium | spec. | 31090 | | | | | |
| Corynebacterium | spec. | 31090 | | | | | |
| Corynebacterium | spec. | 31090 | | | | | |
| Corynebacterium | spec. | 15954 | | | | | |
| Corynebacterium | spec. | 21857 | | | | | |
| Corynebacterium | spec. | 21862 | | | | | |
| Corynebacterium | spec. | 21863 | | | | | |

| | | | | | | | |
|---|---|---|---|---|---|---|---|
| - ATCC:: American Type Culture Collection, Rockville, MD, USA - FERM:: Fermentation Research Institute, Chiba, Japan - NRRL:: ARS Culture Collection, Northern Regional Research Laboratory, Peoria, IL, USA - CECT:: Coleccion Espanola de Cultivos Tipo, Valencia, Spain - NCIMB:: National Collection of Industrial and Marine Bacteria Ltd., Aberdeen, UK - CBS:: Centraalbureau voor Schimmelcultures; Baarn, NL | | | | | | | |

Die so erzeugten Mutanten können dann zur Herstellung von Feinchemikalien verwendet werden oder im Falle von C. diphtheriae für die Herstellung z.B. von Impfstoffen mit abgeschwächten oder nicht-pathogenen Erregern. Unter Feinchemikalien werden verstanden: organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme.

Der Begriff "Feinchemikalie" ist im Fachgebiet bekannt und beinhaltet Moleküle, die von einem Organismus produziert werden und in verschiedenen Industriezweigen Anwendungen finden, wie beispielsweise, jedoch nicht beschränkt auf die pharmazeutische Industrie, die Landwirtschafts-, und Kosmetik-Industrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (beispielsweise Arachidonsäure), Diole (beispielsweise Propandiol und Butandiol), Kohlenhydrate (beispielsweise Hyaluronsäure und Trehalose), aromatische Verbindungen (beispielsweise aromatische Amine, Vanillin und Indigo); Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme Polyketide (Cane *et al*. (1998) *Science* 282: 63-68), und sämtliche anderen von Gutcho (1983) in *Chemicals* by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### A. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe beispielsweise Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthesen mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, beispielsweise Bakterien, ist gut charakterisiert worden (für einen Überblick der bakteriellen Aminosäure-Biosynthese und ihrer Regulation, s.. Umbarger, H.E. (1978) Ann. Rev. Biochem. 47: 533 - 606). Glutamat wird durch reduktive Aminierung von α-Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der-Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet. (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfstoffe. (Für einen Überblick über die Struktur, Aktivität und die industriellen Anwendungen dieser Verbindungen siehe beispielsweise Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (beispielsweise Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATP-getriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (beispielsweise Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die beispielsweise als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (beispielsweise ATP oder GTP) und für Chemikalien selbst, werden gewöhnlich als Geschmacksverstärker verwendet (beispielsweise IMP oder GMP) oder für viele medizinische Anwendungen (siehe beispielsweise Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe beispielsweise Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, beispielsweise Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. beispielsweise Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Dieses Vorgehen kann in analoger Weise auch mit anderen Bakterien durchgeführt werden.

### Beispiel 1: Präparation der genomischen DNA aus Bacillus amyloliquefaciens ATCC 23844

Eine Kultur von *B. amyloliquefaciens* ATCC 23844 wurde in Erlenmeyerkolben mit LB-Medium über Nacht bei 37°C angezogen. Die Bakterien wurden danach durch Zentrifugation pelletiert. 1 g feuchtes Zellpellet wurde in 2 mL Wasser resuspendiert und davon 260 µL in blaue Matrixröhrchen der Fa. Hybaid, #RYM-61111 (Genom Star Kit, #GC-150) überführt. In diese Röhrchen wurde bereits vorgelegt: 650 µL Phenol (äquilibriert mit TE-Puffer, pH 7,5); 650 µL Puffer 1 aus o. a. Kit; 130 µL Chloroform. Die Zellen wurden im Ribolyser (Fa. Hybaid, #6000220/110) für 15 sec bei Rotationseinstellung 4,0 aufgeschlossen und danach bei 4°C und 10.000 upm 5 min lang zentrifugiert. 650 µL des Überstandes wurde dann in 2,0 mL Eppendorf-Gefässe überführt und mit 2 µL RNAse (10 mg/mL) versetzt. Die Inkubation wurde dann bei 37°C für 60 min durchgeführt. Zu dieser Lösung wurden dann 1/10 Volumen 3M Na-Acetat pH 5,5 und 2 Volumen 100% Ethanol zugegeben und es wurde vorsichtig gemischt. Die DNA wurde dann durch 10minütige Zentrifugation bei 4°C und 13.000 upm präzipitiert. Das Pellet wurde mit 70% Ethanol gewaschen und luftgetrocknet. Nach Trocknung wurde das DNA-Pellet in Wasser aufgenommen und photometrisch vermessen.

### Beispiel 2: PCR-Klonierung des Gens für Levansucrase (sacB) aus Bacillus amyloliquefaciens ATCC 23844

Zur Klonierung des Gens für Levansucrase aus *Bacillus amyloliqaefaciens* (ATCC23844) per PCR können als primer Oligonukleotide verwendet werden, die auf Basis publizierter Sequenzen für Levansucrase (beispielsweise Genbank Eintrag X52988) definiert werden können. Die PCR kann nach Methoden durchgeführt werden, die dem Fachmann wohlbekannt sind und beispielsweise in Sambrook, J. et al. (1989) "Molecular Cloning: A Laboratory Manual", Cold Spring Harbor Laboratory Press oder Ausubel, F.M. et al. (1994) "Current Protocols in Molecular Biology", John Wiley & Sons beschrieben sind. Das Gen für Levansucrase (sacB-Gen), bestehend aus der das Protein kodierenden Sequenz sowie 17 bp 5' (Ribosomenbindungsstelle) der kodierenden Sequenz kann im Verlauf der PCR mit terminalen Schnittstellen für Restriktionsendonukleasen (beispielsweise BamHI) versehen werden und anschließend kann das PCR-Produkt in geeignete Vektoren (wie das E. coli Plasmid pUC18) kloniert werden, die über die geeigneten Schnittstellen für Restriktionsendonukleasen verfügen. Diese Methode der Klonierung von Genen per PCR ist dem Fachmann bekannt und beispielsweise in Sambrook, J. et al. (1989) "Molecular Cloning: A Laboratory Manual", Cold Spring Harbor Laboratory Press oder Ausubel, F.M. et al. (1994) "Current Protocols in Molecular Biology", John Wiley & Sons beschrieben. Durch Sequenzanalyse (wie in Beispiel 3 beschrieben) kann nachgewiesen werden, dass das sacB-Gen aus B. *amyloliquefaciens* mit der bekannten Sequenz kloniert wurde. Zur PCR-Reaktion wurden folgende Primer eingesetzt:

### Beispiel 3: Testung der sacB-vermittelten Saccharose-Sensitivität in Corynebacterium glutamicum ATCC13032

Das sacB Gen aus *B. amyloliquefaciens* wurde zunächst unter die Kontrolle eines heterologen Promotors gebracht. Zu diesem Zweck wurde der tac-Promotor aus E. coli durch PCR-Methoden, wie in Beispiel 2 beschrieben, kloniert. Dazu kamen folgende Primer zum Einsatz:

Der tac-Promotor und das sacB Gen wurden dann über die gemeinsame Restriktionsendonuklease-Schnittstelle NotI fusioniert und mittels der AspI und SpeI Schnittstellen in einen shuttle-Vektor kloniert, der sowohl in *E. coli*, als auch in *C*. *glutamicum* replikationsfähig ist und Kanamycin-Resistenz vermittelt. Nach DNA-Transfer in *C*. *glutamicum* (siehe beispielsweise WO 01/02583) und Selektion Kanamycin-resistenter Kolonien wurden etwa 20 dieser Kolonien parallel auf Agarplatten umgestrichen, die entweder 10% Saccharose oder keine Saccharose enthielten. Für diese Selektion geeignet waren CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH, pro Platte: 4 µL IPTG 26%ig), die bei 30°C inkubiert worden sind. Klone mit exprimiertem sacB Gen waren über Nacht nur auf Saccharose-freien Platten angewachsen.

### Beispiel 4: Inaktivierung des ddh Gens aus Corynebacterium glutamicum

Man kann einen beliebigen Sequenzabschnitt am 5'-Ende des ddh-Gens von C. glutamicum (Ishino et al. (1987) Nucleic Acids Res. 15, 3917) und einen beliebigen Sequenzabschnitt am 3'-Ende des ddh-Gens mit bekannten Methoden per PCR amplifizieren. Man kann die beiden PCR-Produkte mit bekannten Methoden derart fusionieren, dass das resultierende Produkt kein funktionales ddh-Gen ergibt. Man kann diese inaktive Form des ddh-Gens, sowie das sacB Gen aus B. amyloliquefaciens in pSL18 (Kim, Y. H. & H.-S. Lee (1996) J. Microbiol. Biotechnol. 6, 315-320) klonieren und so den Vektor pSL18sacBaΔddh erhalten. Die Vorgehensweise ist dem Fachmann geläufig. Der Transfer dieses Vektors in Corynebacterium ist dem Fachmann bekannt und ist beispielsweise möglich durch Konjugation oder Elektroporation.

Die Selektion der Integranten kann mit Kanamycin erfolgen, die Selektion auf den "*pop-out*" kann erfolgen wie in Beispiel 2 beschrieben. Die Inaktivierung des ddh-Gen kann beispielsweise durch fehlende Ddh-Aktivität gezeigt werden. Ddh-Aktivität kann nach bekannten Methoden (siehe z.B. Misono et al. (1986) Agric.Biol.Chem. 50, 1329-1330) gemessen werden.

## Patentansprüche

1. Plasmidvektor, der in Bakterien-Stämmen der Gattung Brevibacterium oder Corynebacterium nicht repliziert, mit folgenden Komponenten:
a) einen Replikationsursprung für *E. coli,*
b) einen oder mehrere genetische Marker,
c) optional einen Sequenzabschnitt, der den Transfer von DNA durch Konjugation ermöglicht (mob),
d) einen Sequenzabschnitt, der homolog zu Sequenzen der Bakterienstämme der Gattung Brevibacterium oder Corynebakterium ist und in diesen Bakterienstämmen homologe Rekombination vermittelt,
e) das sacB Gen aus *B. amyloliquefaciens* unter der Kontrolle eines Promotors.

2. Plasmidvektor nach vorhergehendem Anspruch, wobei der genetische Marker eine Antibiotika Resistenz vermittelt.

3. Plasmidvektor nach einem der vorhergehenden Ansprüche, wobei der Promotor heterolog ist.

4. Plasmidvektor nach einem der vorhergehenden Ansprüche, wobei die Komponente c) vorhanden ist.

5. Plasmidvektor nach einem der vorhergehenden Ansprüche, wobei die Antibiotoka Resistenz eine Kanamycin, Chloramphenicol, Tetrazyklin oder Ampicillin Resistenz ist.

6. Plasmidvektor nach einem der vorhergehenden Ansprüche, wobei der heterologe Promotor aus E. coli oder C. glutamicum stammt.

7. Plasmidvektor nach einem der vorhergehenden Ansprüche, wobei der heterologe Promotor der tac-Promotor ist.

8. Verfahren zur *marker-freien Mutagenese* in *gram-positiven Bakterien-*Stämmen aus der Gattung Brevibacterium oder Corynebacterium umfassend folgende Schritte:
a) zur Verfügung stellen eines Vektors nach Anspruch 1,
b) Transfer des Vektors in ein gram-positives Bakterium der Gattung Brevibacterium oder Corynebacterium
c) selektieren auf einen oder mehrere genetische Marker
d) selektieren eines oder mehrerer Klone von transfizierten gram-positiven Bakterien, indem die transfizierten Klone in einem Saccharose-haltigem Medium kultiviert werden.

9. Verfahren nach Anspruch 8, wobei der DNA-Transfer durch Konjugation oder Elektroporation geschieht.

10. Bakterium, erhältlich nach einem Verfahren der Ansprüche 8 bis 9 bis zu Schritt c).

## Claims

1. A plasmid vector which does not replicate in bacterial strains of the genus Brevibacterium and Corynebacterium, having the following components:
a) an origin of replication for *E. coli*,
b) one or more genetic markers,
c) optionally a sequence section which enables DNA transfer by conjugation (mob),
d) a sequence section which is homologous to sequences of the bacterial strains of the genus Brevibacterium or Corynebacterium and mediates homologous recombination in said bacterial strains,
e) the sacB gene from *B. amyloliquefaciens* under the control of a promoter.

2. The plasmid vector according to the preceding claim, where the genetic marker mediates an antibiotic resistance.

3. The plasmid vector according to either of the preceding claims, where the promoter is heterologous.

4. The plasmid vector according to any of the preceding claims, where component c) is present.

5. The plasmid vector according to any of the preceding claims, where the anibiotics resistance is a kanamycin, chloramphenicol, tetracycline or ampicillin resistance.

6. The plasmid vector according to any of the preceding claims, where the heterologous promoter originates from E. coli or C. glutamicum.

7. The plasmid vector according to any of the preceding claims, where the heterologous promoter is the tac promoter.

8. A method for the *marker-free* mutagenesis in *Gram-positive bacterial strains* from the genus Brevibacterium or Corynebacterium comprising the following steps:
a) provision of a vector according to claim 1,
b) transfer of the vector into a Gram-positive bacterium of the genus Brevibacterium or Corynebacterium
c) selection for one or more genetic markers
d) selection of one or more clones of transfected Gram-positive bacteria by cultivating the transfected clones in a sucrose-containing medium.

9. The method according to claim 8, where the DNA transfer takes place by conjugation or electroporation.

10. A bacterium obtainable by a method of claims 8 to 9 as far as step c).

## Revendications

1. Vecteur de type plasmide, qui ne réplique pas dans des souches bactériennes du genre Brevibacterium ou Corynebacterium, présentant les composants suivants :
a) une origine de réplication pour *E*. *coli,*
b) un ou plusieurs marqueurs génétiques,
c) éventuellement un fragment de séquence qui permet le transfert d'ADN par conjugaison (mob),
d) un fragment de séquence qui est homologue aux séquences des souches bactériennes du genre Brevibacterium ou Corynebacterium et qui procure dans ces souches bactériennes une recombinaison homologue,
e) le gène sacB de *B*. *amyloliquefaciens* sous le contrôle d'un promoteur.

2. Vecteur de type plasmide suivant la revendication précédente, dans lequel le marqueur génétique procure une résistance aux antibiotiques.

3. Vecteur de type plasmide suivant les revendications précédentes, dans lequel le promoteur est hétérologue.

4. Vecteur de type plasmide suivant l'une des revendications précédentes, dans lequel le composant c) est présent.

5. Vecteur de type plasmide suivant l'une des revendications précédentes, dans lequel la résistance aux antibiotiques est une résistance à la kanamycine, au chloramphénicol, à la tétracycline ou à l'ampicilline.

6. Vecteur de type plasmide suivant l'une des revendications précédentes, dans lequel le promoteur hétérologue provient d'*E. coli* ou de *C. glutamicum*.

7. Vecteur de type plasmide suivant l'une des revendications précédentes, dans lequel le promoteur hétérologue est le promoteur tac.

8. Procédé de mutagénèse exempte de marqueur dans des souches bactériennes Gram-positives du genre Brevibacterium ou Corynebacterium, comprenant les étapes suivantes :
a) rendre disponible un vecteur suivant la revendication 1,
b) transférer le vecteur dans une bactérie Gram-positive du genre Brevibacterium ou Corynebacterium,
c) sélectionner sur un ou plusieurs marqueurs génétiques,
d) sélectionner un ou plusieurs clones de bactéries Gram-positives transfectées, en mettant le clone transfecté en culture dans un milieu contenant du saccharose.

9. Procédé suivant la revendication 8, dans lequel le transfert d'ADN a lieu par conjugaison ou électroporation.

10. Bactérie que l'on peut obtenir suivant un procédé selon les revendications 8 et 9, jusqu'à l'étape c).
